# EUROPEAN PATENT SPECIFICATION

(11) **EP 0 739 421 B1**
(45) Date of publication and mention of the grant of the patent: **19.08.1998**
(21) Application number: 95905195.4
(22) Date of filing: 10.01.1995
(51) Int. Cl.: C12Q 1/68

(54) **HYDRIDISATION ASSAY**
HYBRIDISIERUNGSVERFAHREN
DOSAGE PAR HYBRIDATION

(30) Priority: 10.01.1994 GB 9400300
(43) Date of publication of application: 30.10.1996
(73) Proprietor: CELSIS INTERNATIONAL plc, Cambridge CB4 4FX (GB)
(72) Inventor: GRANT, Peter Leonard, Needingworth Cambridgeshire PE17 3UE (GB); FORSTER, Simon James, Ely Cambridgeshire CB7 4BL (GB); FORSTER, Simon James, Ely Cambridgeshire CB7 4BL (GB)
(74) Representative: Perry, Robert Edward
(86) International application number: GB9500048
(87) International publication number: WO9518867

(56) References cited:
- EP-A- 0 146 039
- WO-A-93/17128

## Description

### Field of the Invention

This invention relates to assay methods, and reagents for use therein, for the detection and quantitation of analytes by means of nucleic acid probes. The analytes are specific nucleic acid sequences. In particular, this invention relates to an homogeneous assay whereby such analytes are detected and quantitated in solution.

### Background of the Invention

The detection of the presence of specific nucleic acid sequences in a given sample, by means of nucleic acid probes which bind ("hybridize") to the said sequences is well known in the art. The classical format for probe hybridisation is the Southern blot. A variant is the dot-blot or slot-blot. In both cases, the immobilisation of the target on a solid phase means that excess unhybridised probe can be easily removed by washing.

More recent systems incorporate hybridisation in the liquid phase, which is more rapid. Since the characteristics of most labelling systems still require the removal of any unhybridised probe, in many test formats solution hybridisation is followed by capture of the hybrids onto a solid phase.

A variety of dual probe "sandwich" formats have been described, in which the target sequence forms a link between a labelled "reporter" probe and an immobilised "capture" probe. The reporter probe becomes immobilized only in the presence of the target. Increasingly, however, the need has been seen for homogeneous systems, where a signal can be generated in the liquid phase only if probe:target hybrids are formed. In other words, no removal of excess unbound probe is necessary.

EP-A-0146039 describes a homogeneous assay system based on enzyme channelling. Antibody to double-stranded DNA is conjugated to two different enzymes and both types of conjugate are added to the hybridisation mixture; if probe:target hybrids are formed, the two enzyme labels are brought close together. The hybridisation mixture also contains a complete substrate for the first enzyme but not for the second. One suitable enzyme pair is glucose oxidase and peroxidase; a high local concentration of hydrogen peroxide is generated by the glucose oxidase, allowing the peroxidase to form a coloured product. If both antibodies were free in solution, the concentration of hydrogen peroxide seen by the peroxidase would be negligible and no colour would be formed. Heller et al, in Kinsgbury, D.T. & Falkow, S. eds., Rapid Detection and Identification of Infectious Agents, Academic Press, New York (1985), describe a similar concept, in which two separate probes are used whose target sequences are adjacent. Both have labels which are fluorescent; the emission wavelength of one label being the appropriate excitation wavelength of the other. When the two labels are in close proximity, energy transfer and hence emission by the second label are highly efficient. Again, if the two probes are free in solution, emission from the second label is negligible.

A fluorescent homogeneous test using only one probe has also been developed; see Arnold, Clin. Chem. 35:1588-1594 (1989) and EP-A-0309230. The label is an acridinium ester containing a cleavable bond. Cleavage of this bond renders the label non-fluorescent. The structure of the probe:target hybrid protects this bond from cleavage. To perform the test, probe is added to target in solution and allowed to hybridise. A cleavage reagent is then added. The level of fluorescence remaining after the cleavage treatment is a direct measure of the degree of hybridisation which has occurred and hence of the amount of target.

Homogeneous tests such as those just described, which are extremely simple from the point of view of the user, are likely to become more common.

WO-A-9317128 discloses a method for the detection of chromosomal structure and rearrangements. In this method, labelled DNA probes are contacted with the chromosomes under hybridising conditions, and the labels are then bound by antibodies to different enzymes which, in combination, provide a signal that can be detected by optical means. The target sequence is immobilised. The enzymic reactions are allowed to run only after removal of excess probe.

### Summary of the Invention

The present invention provides a dual probe homogeneous assay as defined in claim 1 wherein the sequences recognised by the two probes are closely adjacent to each other in the linear sequence of the target, and wherein each probe is labelled, by any of a variety of means, with a different enzyme. Labelling is such that, when hybridisation to the target nucleic acid sequence occurs, the two enzyme labels are brought into close proximity. The enzymes form a channelling pair; that is, a product of the first enzymic reaction (for which a complete substrate mixture is initially provided) is a necessary component of the substrate mixture for the second enzymic reaction.

The present invention provides various important features. The manipulations which it is necessary for the operator to carry out are minimal. The probe(s), the sample to be tested and all the components necessary for the generation of the appropriate signal can be combined in a single liquid mixture. If the test sample contains the target DNA sequence, signal is generated. If it does not, no signal is generated. The signal can be proportional to the amount of target present in the sample, and the signal can be quantitated. The test reveals not only whether or not the target is present in the sample, but also, if it is present, how much is present. Tests which employ dual probes have the further advantage that the requirement for two hybridisation events improves specificity.

### Description of the Invention

The assay is carried out in the following manner: a liquid mixture which contains the two probes, a complete substrate mixture for the first enzyme and an incomplete substrate mixture for the second enzyme (lacking only the component which is a product of the first enzymic reaction), is added to a liquid mixture containing the target, under conditions such that specific hybridization of the two probes to their adjacent targets is favoured. In the absence of the target sequence, all the available probe molecules, and consequently the two enzyme labels also, remain dispersed throughout the entire volume of the liquid mixture. Although the first enzymic reaction still proceeds, the effective concentrations of the products of this reaction relative to the concentration of the second enzyme remain low, and product (signal) formed by the second enzyme remains negligible. In the event of the presence of the target sequence, hybridization occurs and some of the available probe molecules, and hence the two enzyme labels, are brought into close proximity. A high local concentration of the products of the first enzymic reaction is now formed in the micro-environment of the two co-localised probes. This allows the product of the second enzymic reaction, which constitutes the primary signal, to be formed. Any probes in excess, remaining unhybridized in the presence of target, behave as they would do in the absence of target. Thus there is no need to perform any additional manipulation or separation step to remove unhybridized probes, as their contribution to the signal generated is negligible.

A further feature of the invention is the inclusion, in the mixture of substrates, of a competitive inhibitor of the second enzymic reaction. This provides a further enhancement of the increase in the reaction rate of the second enzyme system when target is present. When the two probes are not co-localized by hybridization to the target, the first enzymic reaction still takes place, and the product of that reaction which is necessary to allow the second enzymic reaction to occur is produced. The concentration of this product available to the second enzyme is, however, low. The presence of a substance which competes with this product for the active site of the second enzyme will further reduce the rate of formation of the product(s) of the second enzyme reaction. When the two probes are co-localized by hybridization to the target, the concentration of the product of the first reaction which is available for the second enzyme is effectively much higher in the micro-environment of the co-localized probes, whereas the concentration of the inhibitor remains the same as in the case where the probes are not co-localized. Thus the difference between the rates of the second reaction, which determines the level of production of the signal which is to be measured, in the two cases (probes not co-localized or probes co-localized), and which is inherent in the basic embodiment of the invention, is further increased by the presence of a competitive inhibitor of the second enzyme reaction.

In a further embodiment of the invention, an alternative method for attaching the enzymes to the probes is employed. In this embodiment, each probe has a sequence additional to that which is substantially complementary to the target. In the case of the first probe this additional sequence is at the 3'-end and in the case of the second probe this additional sequence is at the 5'-end, such that when the probes are hybridized to their targets, the said additional sequences are in close proximity to each other. Two further short oligonucleotides are present in the assay, each being complementary to the additional sequences, such that short double-stranded sequences are created. These double-stranded sequences are designed to be capable of being recognised by sequence-specific DNA-binding proteins. The appropriate DNA-binding proteins are also included in the assay mixture, formulated in such a way that each one forms part of the first or the second enzyme of the channelling pair. The linkage is covalent. This may be achieved either by expressing the DNA-binding protein and the enzyme as a fusion protein, or by using specific chemical cross-linking reagents, both these being achievable by means which are well known in the art.

Examples of enzyme channelling pairs are most conveniently grouped into several categories according to the second enzyme of the pair.

One group comprises enzyme pairs where the second enzyme of the pair is firefly luciferase. First enzymes in this group include alkaline phosphatase, β-galactosidase, carboxypeptidases A and B, carboxylic esterase and arylsulphatase.

Another group comprises enzyme pairs where the second enzyme of the pair is horse-radish peroxidase. First enzymes in this group include glucose oxidase, xanthine oxidase and D-amino acid oxidase.

It is especially preferred that the second enzyme is horse-radish peroxidase and the first enzyme is glucose oxidase or xanthine oxidase or D-amino acid oxidase; or, the second enzyme is firefly luciferase and the first enzyme is alkaline phosphatase or β-galactosidase.

DNA-binding proteins suitable for use in the invention include lac operator binding protein, *Anaboena* bifA gene product, MAT-α-2 gene product or *Staphylococcus aureus* repN gene product. There is no restriction on particular combinations of the DNA-binding proteins with the two enzymes of a channelling pair, except that the DNA-binding protein part of the first enzyme of the channelling pair must be different from the DNA-binding protein part of the second enzyme of the channelling pair.

### Example

A detailed description of one embodiment of the invention will now be given by way of example. In this description, "conjugate" refers to a macromolecular assemblage comprising enzyme label covalently linked to oligonucleotide probe, "first probe" refers to that probe of the pair which carries enzyme label at its 3'-end, "second probe" refers to that probe of the pair which carries enzyme label at its 5'-end, "first enzyme" refers to that enzyme for which a complete substrate mixture is provided and "second enzyme" refers to that enzyme for which the substrate mixture is initially incomplete, the missing component being supplied by the reaction of the first enzyme with its complete substrate. Note that the first enzyme can be attached to the first or second probe; if the first enzyme is attached to the first probe then the second enzyme must be attached to the second probe; if the first enzyme is attached to the second probe then the second enzyme must be attached to the first probe.

In the embodiment to be described the channelling enzyme pair is alkaline phosphatase (first enzyme) and firefly luciferase (second enzyme). The first enzyme is attached to the first probe and the second enzyme is attached to the second probe. The substrate for the first enzyme is luciferin-O-phosphate, which is not a substrate for the production of bioluminescence mediated by luciferase, but is converted by alkaline phosphatase to luciferin, which is such a substrate.

Conjugates were prepared of the first enzyme and the first probe, and of the second enzyme and the second probe. Procedures for the conjugation of enzymes to oligonucleotide probes are well known in the art. The first probe was synthesized with an additional modified base at the 3'-end of the oligonucleotide, this base carrying a primary amine at the end of an alkyl chain; that is, the probe carried at its 3'-end an ω-aminoalkyl group. Similarly the second probe was synthesized with an additional modified base at the 5'-end of the oligonucleotide, this base again carrying a primary amine at the end of an alkyl chain; that is, the probe carried at its 5'-end an ω-aminoalkyl group. Methods for the chemical synthesis of such modified probes are well known to those skilled in the art.

A conjugate comprising the first probe linked through the modified 3'-end to alkaline phosphatase was prepared and purified using a reagent kit (E-Link kit, Cambridge Research Biochemicals). Other such kits are available and suitable.

A conjugate comprising the second probe linked through the modified 5'-end to firefly luciferase was prepared and purified thus: 70 µg of oligonucleotide was dissolved in 25 µl of 0.1M sodium borate, pH 9.3. To this was added 10 mg phenylene diisothiocyanate dissolved in 0.5 ml N,N'-dimethylformamide. The mixture was incubated for 2 hours at room temperature in the dark with continuous mixing; 3 ml of butan-1-ol and 3 ml of water were then added and mixed thoroughly. The mixture was centrifuged at 1500 rpm for 3 minutes. The upper aqueous phase was discarded. An equal volume of butan-1-ol was added and mixed thoroughly. The mixture was again centrifuged and the upper aqueous layer was removed and discarded. Extractions with an equal volume of butan-1-ol were continued until the volume of the lower phase was reduced to less than 50 µl. The activated oligonucleotide was then dried completely under vacuum. 1 mg of luciferase in 50 µl 0.1 M sodium borate buffer, pH 9.3, was added and the mixture was incubated at 4°C for 5 hours. The conjugate was then purified by anion-exchange HPLC. The reaction mixture was loaded onto a TSK DEAE-5PW column equilibrated in 0.1 M Tris, pH 8.4, and eluted with a gradient of NaCl in the same buffer. Fractions containing luciferase-oligonucleotide conjugate were identified by methods well known in the art and pooled.

The target sequence is preferably in single-stranded form. Procedures for generating single-stranded DNA are well known to those skilled in the art. By way of example only, single-stranded DNA may be generated by exonuclease III digestion, by asymmetric PCR or by PCR with one labelled primer, followed by capture onto a suitable solid phase mediated by the label. The unlabelled strand can then be released by denaturation.

The assay is carried out as follows: the assay buffer contains 0.1 mM luciferin-O-phosphate, 2.6 mM ATP, 6 mM MgCl₂, 3.4 mM DTT, 0.54 mM EDTA in 0.1 M Tris, pH 8.0. To 100 µl of this, target (1-2 µg) is added, followed by probes in the ratio 1:2 (alkaline phosphatase-labelled probe: luciferase-labelled probe) . Light output is measured at 1 minute intervals for 10 minutes, using any suitable luminometer.

## Claims

1. A homogeneous assay for a target nucleotide sequence in a sample, which comprises bringing into contact with the sample first and second nucleotide probes respectively labelled with first and second enzymes of a channelling pair, i.e. a product of the first enzymic reaction is a necessary component of the substrate mixture for the second enzymic reaction, and wherein the probes hybridize at closely adjacent sites on the target sequence in such a way that the two enzyme labels are brought into juxtaposition, and introducing the materials, excluding said product/component, which allow the enzymic reactions to run, thereby generating a detectable signal in the presence of the target but not in its absence.

2. An assay according to claim 1, wherein the first enzyme is alkaline phosphatase or β-galactosidase.

3. An assay according to claim 1, wherein the first enzyme is glucose oxidase, xanthine oxidase or D-amino-acid oxidase.

4. An assay according to claim 1 or claim 2, wherein the second enzyme is luciferase.

5. An assay according to claim 1 or claim 3, wherein the second enzyme is peroxidase.

6. An assay according to any preceding claim, wherein each probe has, with respect to that which is substantially complementary to the target sequence, additional sequence which can hybridise with a further oligonucleotide to form a length of double-stranded DNA with sequence capable of being bound by a sequence-specific DNA binding protein, the respective additional sequences, further oligonucleotides and binding proteins being different for each probe.

7. An assay according to claim 6, which comprises introducing the further oligonucleotides and the DNA-binding proteins, each DNA-binding protein being itself bound to enzyme label.

8. An assay according to any preceding claim, which additionally comprises introducing a competitive inhibitor of the second enzyme of the channelling pair, thereby enhancing the signal.

## Patentansprüche

1. Homogener Assay für eine Target-Nukleotidsequenz in einer Probe, umfassend das Kontaktieren mit der Probe von ersten und zweiten Nukleotid-Sonden, die mit ersten bzw. zweiten Enzymen eines kanalbildenden Paares, d.h. ein Produkt der ersten Enzym-Reaktion ist eine notwendige Komponente der Substrat-Mischung für die zweite Enzym-Reaktion, markiert sind, und wobei die Sonden an eng benachbarten Stellen auf der Target-Sequenz auf solche Weise hybridisieren, daß die beiden Enzym-Marker in Nachbarschaft zueinander gebracht werden, und das Einführen der Materialien mit Ausnahme des Produkts/der Komponente, die ein Ablaufen der Enzym-Reaktionen ermöglichen, wodurch in Anwesenheit des Targets ein meßbares Signal erzeugt wird, aber nicht in dessen Abwesenheit.

2. Assay nach Anspruch 1, in welchem das erste Enzym alkalische Phosphatase oder β-Galactosidase ist.

3. Assay nach Anspruch 1, in welchem das erste Enzym Glucoseoxidase, Xanthinoxidase oder D-Aminosäureoxidase ist.

4. Assay nach Anspruch 1 oder Anspruch 2, in welchem das zweite Enzym Luciferase ist.

5. Assay nach Anspruch 1 oder Anspruch 3, in welchem das zweite Enzym Peroxidase ist.

6. Assay nach irgendeinem vorangehenden Anspruch, in welchem jede Sonde hinsichtlich derjenigen, die im wesentlichen komplementär zur Target-Sequenz ist, zusätzliche Sequenz aufweist, die mit einem weiteren Oligonukleotid unter Bildung eines Stücks doppelsträngiger DNA mit Sequenz, die an ein Sequenz-spezifisches DNA-bindendes Protein gebunden werden kann, hybridisieren kann, wobei für jede Sonde entsprechende zusätzliche Sequenzen, weitere Oligonukleotide und bindende Proteine verschieden sind.

7. Assay nach Anspruch 6, umfassend die Einführung der weiteren Oligonukleotide und der DNA-bindenden Proteine, wobei jedes DNA-bindende Protein selbst an Enzym-Marker gebunden ist.

8. Assay nach irgendeinem vorangehenden Anspruch, weiter umfassend die Einführung eines konkurrierenden Inhibitors des zweiten Enzyms des kanalbildenden Paares, wodurch man das Signal verstärkt.

## Revendications

1. Un test homogène pour une séquence cible nucléotidique dans un échantillon, qui consiste à mettre en contact avec l'échantillon une première et une seconde sonde nucléotidique respectivement marquées avec un premier et un second enzyme d'une paire complémentaire, où le produit de la première réaction enzymatique est un composant nécessaire du mélange de substrat pour la seconde réaction enzymatique, et dans lequel les sondes hybrident sur des sites directement adjacents de la séquence cible de manière à ce que les deux marqueurs enzymatiques sont directement juxtaposés, consistant à introduire les matériaux permettant aux réactions enzymatiques de se produire à l'exception dudit produit/composant, produisant par là un signal détectable en présence de la cible, mais non en son absence.

2. Un test selon la revendication 1, dans lequel le premier enzyme est la phosphatase alcaline ou la β-galactosidase.

3. Un test selon la revendication 1, dans lequel le premier enzyme est la glucose oxydase, la xantine oxydase ou la D-amino-acide oxydase.

4. Un test selon l'une des revendications 1 ou 2, dans lequel le second enzyme est la luciferase.

5. Un test selon l'une des revendications 1 ou 3, dans lequel le second enzyme est la peroxydase.

6. Un test selon l'une des revendications précédentes, dans lequel chaque sonde comporte, outre la séquence qui est substantiellement complémentaire à la séquence cible, une séquence additionnelle capable de s'hybrider avec un oligonucléotide additionnel pour former une longueur d'ADN double-brins avec une séquence capable d'être liée par une protéine liant l'ADN d'une manière spécifique à la séquence, les séquences additionnelles respectives, les oligonucléotides additionnels et les protéines de liaison étant différents pour chaque sonde.

7. Un test selon la revendication 6, qui comprend l'introduction des oligonucléotides additionnels et des protéines liant l'ADN, chaque protéine liant l'ADN étant elle même liée à un marquage enzymatique.

8. Un test selon l'une des revendications précédentes, qui comprend en outre l'introduction d'un inhibiteur compétitif du second enzyme de la paire complémentaire, par là augmentant le signal obtenu.
